Europäisches Patentamt

European Patent Office

Office européen des brevets

(11) Publication number: **0 388 115 A1**

# EUROPEAN PATENT APPLICATION

(21) Application number: **90302599.7**

(51) Int. Cl.⁵: **C02F 1/50**

(22) Date of filing: **12.03.90**

(30) Priority: **13.03.89 US 322603**
**13.03.89 US 322469**

(43) Date of publication of application:
**19.09.90 Bulletin 90/38**

(84) Designated Contracting States:
**AT BE CH DE DK ES FR GB GR IT LI LU NL SE**

(71) Applicant: **NALCO CHEMICAL COMPANY**
**One Nalco Center**
**Naperville Illinois 60563-1198(US)**

(72) Inventor: **Wiatr, Christopher L.**
**298 Devlin Court**
**Naperville, Illinois 60565(US)**

(74) Representative: **Harrison, David Christopher**
**et al**
**MEWBURN ELLIS 2 Cursitor Street**
**London EC4A 1BQ(GB)**

(54) **Controlling industrial slime.**

(57) A method of attacking and removing microbial slime in slime covered surfaces and maintaining a slime-free surface comprises utilizing the enzymes beta-glucanase or cellulase, or a blend of beta-glucanase, alpha-amylase and protease. Also, beta-glucanase has been found specifically to digest microbial slime and reduce microbial attachment and biofilm. A specific combination of polysaccharide degrading enzymes is a ratio of 2 parts beta-glucanase to 1 alpha-amylase to 1 protease, utilized at a rate of 2-100 parts per million. Broadly, the alpha-amylase must be at least 1 part and the protease may vary from .5 to 1 part.

FIGURE 2:
BIOFILM REMOVAL TEST
SINGLE CYCLE SYNTHETIC H₂O---pH 8.5

CONTROL (BLANK)
BIODISPERSANT
CELLULASE
α-amylase + β-glucane + protease (1:2:1 rat

TIME (HR)

## CONTROLLING INDUSTRIAL SLIME

The present invention relates to enzyme systems for treating microbially produced extracellular polymers, which are present or which build up on surfaces of cooling water towers and in paper-making broke water. Such extracellular polymers plus microbial cells are also known as biofilm or microbial slime.

Microbially produced extracellular polymers can build up, retard heat transfer and restrict water flow through cooling water systems. Controlling slime-forming bacteria by applying toxic chemicals is becoming increasingly unacceptable due to environmental problems. In addition, the efficacy of the toxicants is minimized by the slime itself, since the extracellular polysaccharides enveloping microorganisms are largely impenetrable.

Toxicants cannot adequately control large populations of attached bacteria and they are effective mainly against floating microorganisms. Although surfactants and dispersants which penetrate and help loosen slime can enhance the activity of toxicants, they are nonspecific and may have deleterious effects on the industrial process.

This invention describes the use of certain enzymes and enzyme compositions which have the advantage of being both specific and non-toxic. The approach is designed to (a) enhance the removal of slime where it has formed, (b) prevent the build-up of slime, and (c) improve the efficacy of biocides against sessile bacteria.

Examples of prior art single enzyme formulations are those found in:

US-A-3,773,623 where the slime formulation in industrial water such as white water from pulp and paper mills is retarded by controlling amounts of the enzyme levan hydrolase;

US-A-4,055,467 describes an industrial process whereby slime can be dispersed and prevented by treating said slime with a few ppm of the enzyme Rhozyme HP-150, a pentosanase-hexosanase; and

US-A-3,824,184 describes slime formation controlled by intentionally adding to industrial water the controlled amounts of the enzyme levan hydrolase.

Additionally, US-A-4,684,469 discloses a method of using a two-component biocidal composition suitable for controlling slime. The preparation consists of a biocide and a polysaccharide degrading enzyme.

Methylene-bis-thiocyanate has been preferred as a biocide. Other operable biocides include chlorophenate compounds, such as pentachlorophenates and trichlorophenates; organomercurial compounds, such as phenylmercuric acid; carbamate compounds, such as methyldithiocarbamates, ethylenebisdithiocarbamates, and dimethyldithiocarbamates; carbonate compounds such as cyanodithioimidocarbonates; thiocyanates such as chloroethylene thiocyanate compounds; and other biocides such as bromo-hydroxyacetophenone compounds, benzothiazole compounds, ethylene diamine compounds, nitrilopropionamides, bromopropionamides, bromoacetoxybutenes, bromopropanolaldehyde compounds, bistrichloromethyl sulfones, bimethyl hydantoin compounds, and the like. Mixtures of biocides can also be used.

The biocide methylene-bis-thiocyanate has proven to be particularly effective, as has a combination of dimethyldithiocarbamate and disodium ethylenebisdithiocarbamate.

The disadvantages of the use of biocides to control bacteria are that the biocides constitute toxicants in the system and that pollution problems are ever-present.

According to the present invention the enzymes, cellulase and/or beta-glucanase attack a narrow band of carbohydrate polymers without the broad effects of toxicants. In one aspect of the present invention we increase the range of attack by a composition which includes beta-glucanase and an alpha-amylase along with protease, broadly attaching the carbohydrate polymer and protein surrounding the bacteria. A specific formulation of ratios of the multiple enzyme composition is 2 parts beta-glucanase, 1 part alpha-amylase, and 1 part protease (though the alpha-amylase can be up to 2 but more usually up to slightly over 1 part and the protease may be .5 to 1 part, while the beta-glucanase remains 2 parts), all parts being by weight of the composition.

A preferred composition is 2 parts beta-glucanase, 1 part alpha-amylase and 1 part protease. In the composition cellulase may be partially or completely substituted for beta-glucanase; cellulase may however be used alone.

The glucanase specifically attacks the slime layer surrounding the bacteria. Consequently, the microorganisms become planktonic - harmless in terms of biofilm production - and are rendered susceptible to biocides. Cellulase attacks the beta-linkage between sugar molecules. The enzymes also act to maintain a clean surface.

The concentration of glucanase or cellulase required for effectiveness varies greatly and can depend upon the conditions such as temperature and pH of the water, the microbial count and the type of industrial water being treated. The lower and upper limits of the required concentrations will substantially depend upon the specific enzyme or combination of enzymes used. For example, a concentration of about 1 or 2 parts enzyme to one million parts industrial water in the context of this invention may suffice, or a minimum concentration of 80 or 100 ppm may be required. Normally, however, a dosage of 2 to 10 ppm will suffice.

In contrast to the prior art, the composition embodying the invention is both more specific and non-toxic. It can be said that the present composition has the same target polymers but digests them more efficiently because of the enzyme activities of cellulase or of glucanase in admixture with alpha-amylase, and the protease.

The action of alpha-amylase alone is to attack the alpha-linkage between glucose molecules. It nicks the outside of the slime molecule, so that the beta-glucanase can enter and attack the carbohydrate content. The protease attacks extracellular protein molecules.

Up to this time, enzyme treatment of industrial slime or slime polymer made by bacteria consisted of a single enzyme, for example levanase. Levanase would break down a polymer of levan into its subunits (fructose). However, resistant bacteria would still remain to proliferate. Further applications of levanase were ineffective because the polymer it attacks was no longer present. Other slime polymers would still be there and the bacteria would flourish. Although other enzyme preparations have been used in the marketplace, for example EDC, a levan hydrolyzer (Sunoco), there has been no combination of enzymes that would actually attack polymers made by Pseudomonas bacteria and other bacteria in the field, such as Klebsiella, Acinetobacter, Flavobacterium, Enterobacter, and Aerobacter, which were rich in glucose, mannose and gulose sugars arranged in polymers.

In response to the prior art above, the present inventor has taken a clear culture of Pseudomonas bacteria and made them produce a slime polymer in a low substrate environment. Second, the inventor has taken a composite of microorganisms from the field blended with and grown together both at the laboratory and under field conditions, simulated cooling tower water and utility water.

The results of treatment indicate that the maximum removal of carbohydrate layers from pending bacteria has occurred. Thus utilizing glucanase or cellulase has a superior result, especially as the enzyme utilization was found to be useful against the very prevalent Pseudomonas bacteria.

To assess the composition, a variety of enzymes were utilized in testing against Pseudomonas bacteria. From 42 preparations of enzymes, three types of enzymes were found to be effective on slime produced by Pseudomonas bacteria. First, alpha-amylase was found to attack bacterial slime. Second, protease has been found also to have an effect on bacterial slime. Then it was found that a combination enzyme treatment with amylase, glucanase, and protease was effective in removing the biofilm.

In the drawings:

Figure 1 is a plan view of the biofilm removal reactor system;

Figure 2 is a graph indicating biofilm removal due to biodispersant, cellulase, and a mixture of alpha-amylase, beta-glucanase, protease in a 1:2:1 ratio;

Figure 3 is a plan view of the microbial fouling reactor system;

Figure 4 is a graph showing biofilm mass versus time for glucanase treatment and biodispersant;

Figure 5 is a graph showing pressure drop versus time for glucanase treatment and biodispersant; and

Figure 6 is a graph showing the results of a treatment of enzyme versus biocide in a Microbial Fouling Reactor experiment.

Beta-glucanase can be obtained from many chemical suppliers such as American Cyanamid, Betz, Beckman, Dearborn Chemical, Economics Laboratory, Inc., Merck, Nalco and Vineland Chemical.

## EXAMPLES

### Example I

Preliminary activity screening of about forty enzyme candidates was carried out using slimed microscope slides which were treated with the enzyme candidates in small, stirred, sterile beakers. The test slides were prepared in a slime generation box using a colony isolate of Pseudomonas or a composite of field microorganisms known to produce extracellular polymers in industrial waters. Bacteria were propagated in tryptic soy broth (TSB) and were enumerated on tryptone glucose extract (TGE). Anhydrous dextrose (D-glucose) was used to supplement the TSB nutrient.

Enzyme digestion rates were determined at 1, 2 and 4-hour intervals by assessing biofilm removal from the slides visually. Enzyme candidates showing promising activity in this screening test were explored more fully as below.

Example II

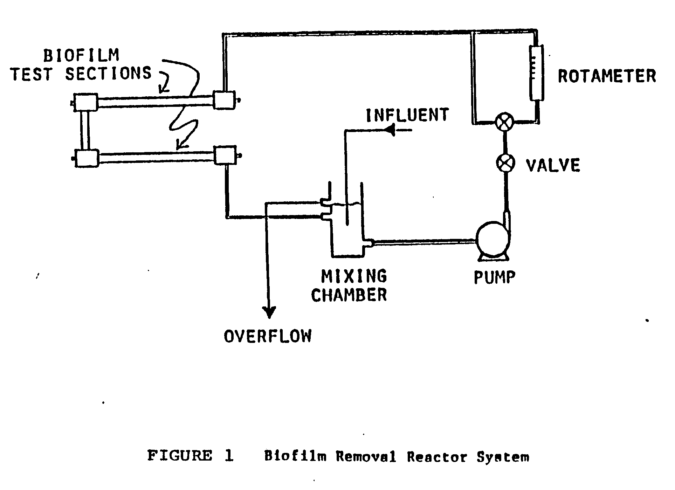

The nine most promising carbohydratases and proteases from the screening test were subjected to further examination using a Biofilm Removal Reactor (BRR) which simulates water-tube fouling in field applications. The reactor is shown schematically in Figure 1. The reactor tubes were first slimed by exposure to slime-forming bacteria in circulated minimal substrate for a 72-hour period.

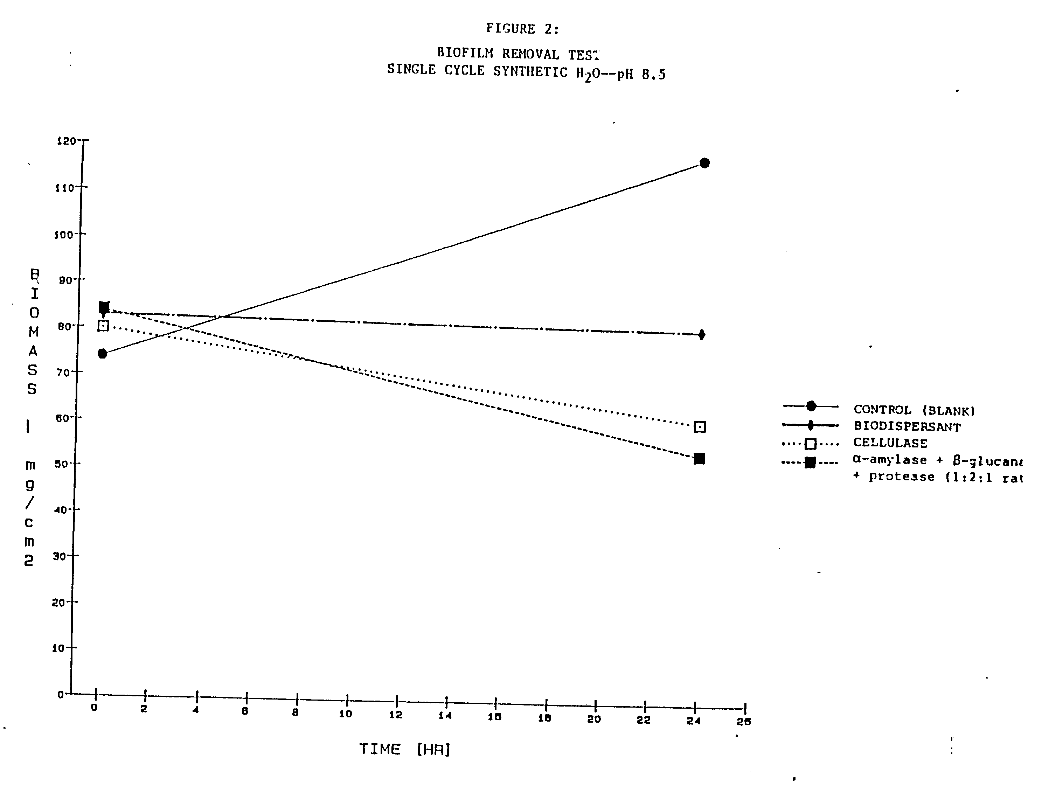

Each of the candidate enzymes was tested in the reactor at a level of 100 ppm for a 24-hour period under the conditions shown in Table 1. The removal of biofilm in the BRR was measured in terms of the percent decrease in biomass resulting from enzyme treatment of the fouled system. The results for a control, a biodispersant, cellulase and the mixed protease-carbohydratase are shown in Figure 2 and Table II. For these tests, the reactor tubes were dried overnight at 60 degrees C and weighed; then cleaned, dried and reweighed to obtain the recorded gravimetric data.

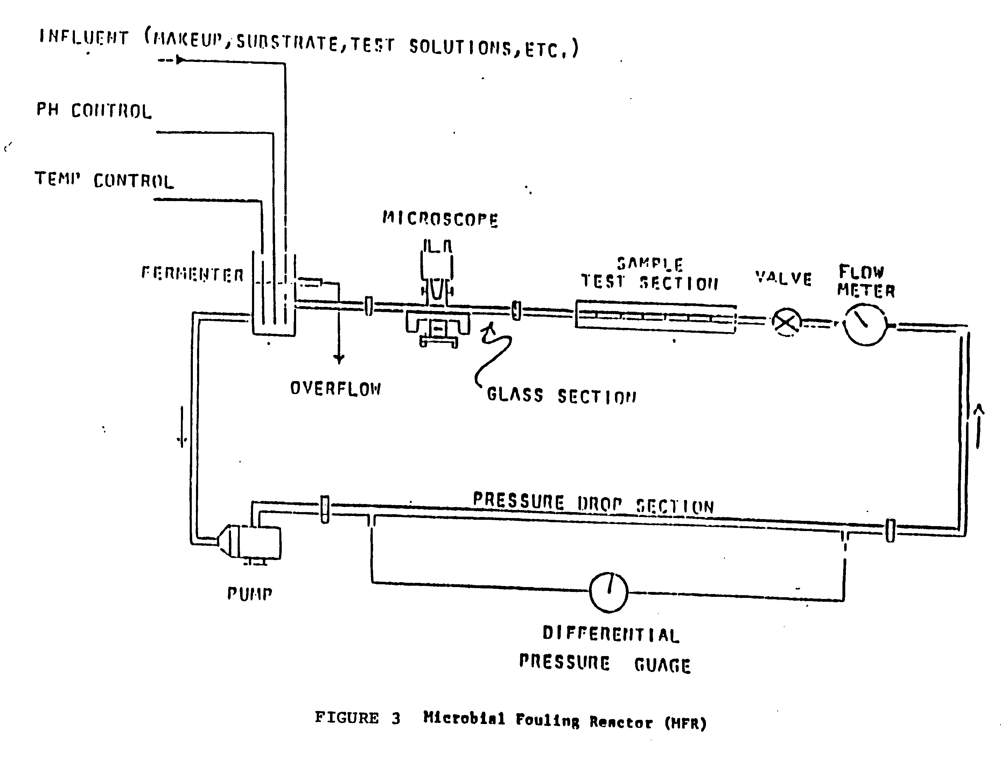

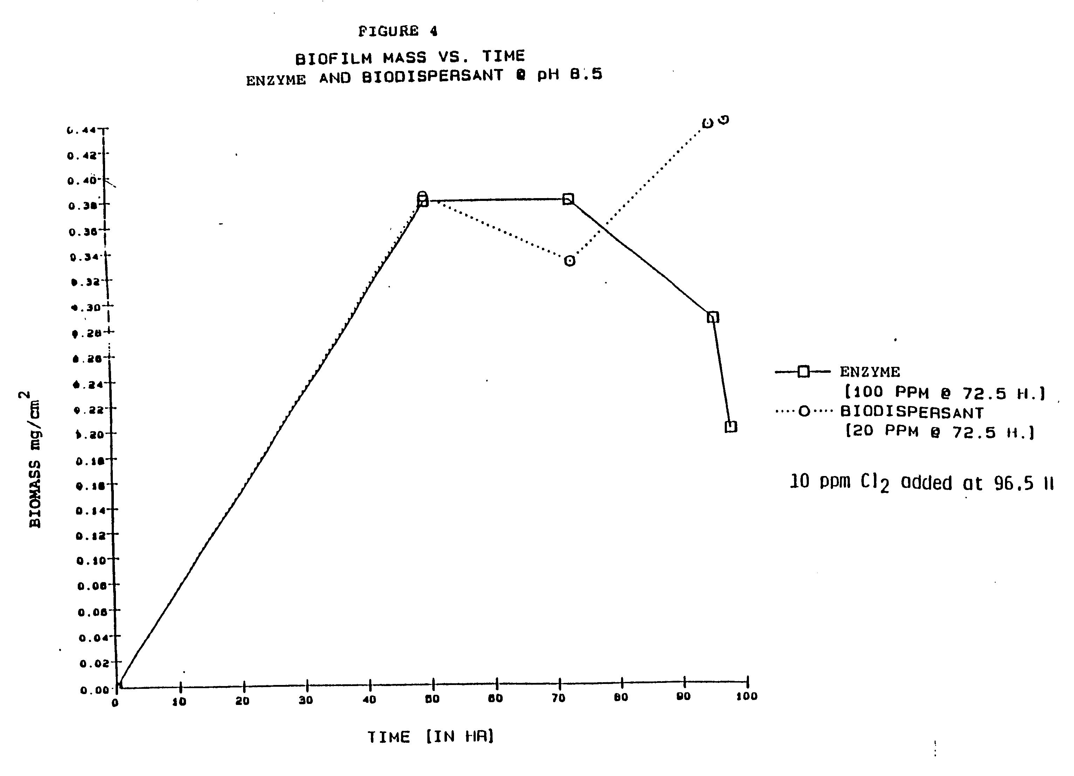

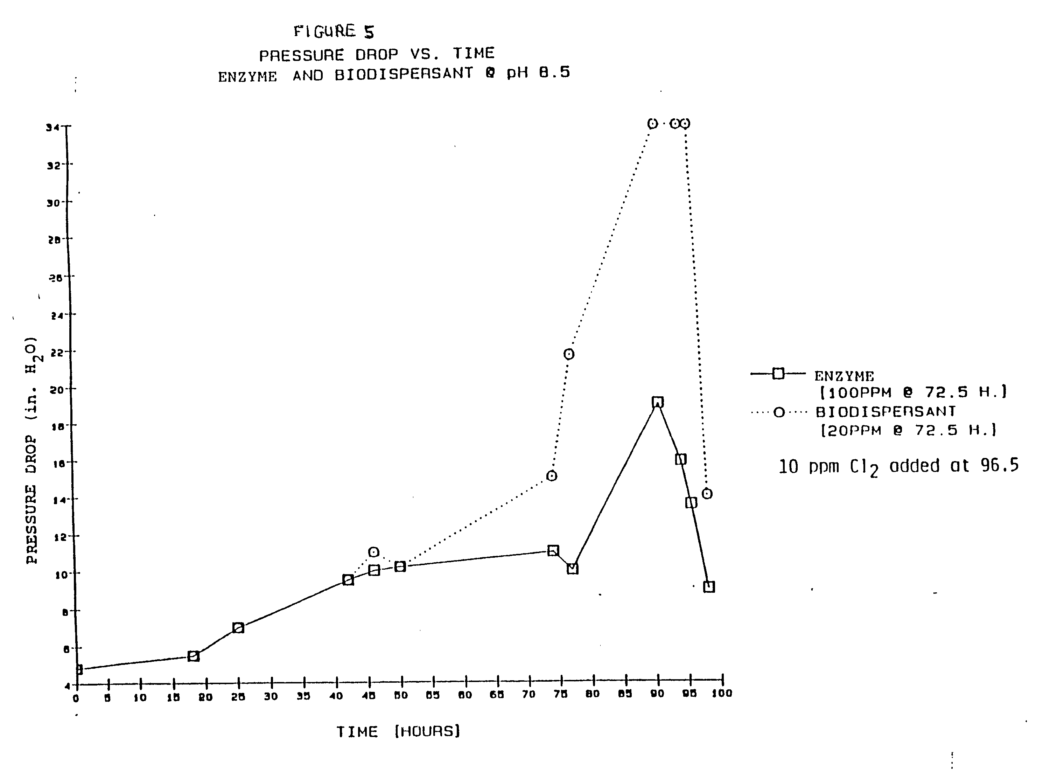

Further tests of these enzymes were conducted in a Microbial Fouling Reactor (MFR), a similar apparatus which also provides for a measure of pressure drop across the slimed reactor tubes as a criterion of fouling. The apparatus is shown in Figure 3 and the experimental conditions are listed in Table I. The experimental procedure for the biomass measurements was generally similar to that used in BRR, above, except that the biomass is measured several times during the course of the experiment. In addition, the effectiveness of the enzyme treatment is measured by the decrease in pressure drop across the slimed tubes of the reactor as well as by visual observation in the slight glass section. Figures 4 and 5 show the results of tests of the mixed glucanase, amylase and protease compared to a polyol biodispersant.

Five of the enzyme preparations tested in the Biofilm Removal Reactor were effective in controlling slime. These are tabulated in Table II with their relative effectiveness. Of these, the glucanase composition was clearly the best performer. This enzyme composite is a combination of one protease and two carbohydratases, namely alpha-amylase and beta-glucanase. It was found to be effective in digesting slime layers produced by cultures of pure and mixed strains of bacteria. A 1:2:1 mixture of alpha-amylase, beta-glucanase and protease is shown in the table to give 37% biomass removal in the time period of the test.

The Biofilm Removal Reactor (Figure 1) results are also depicted in Figure 2. In the biofilm removal experiments, the enzyme cellulase removed 23% of the biomass (62 mg/cm$^2$ after treatment as opposed to 80 mg/cm$^2$ before treatment) in 24 hours. The 1:2:1 combination of alpha-amylase, beta-glucanase, and protease enzymes removed 37% of the biofilm in the same time frame. The control (blank), which was untreated, continued to a 65% increase in biomass. For comparison, a non-enzymatic chemical biodispersant essentially checked overall development of biofilm but did not remove any biomass. Therefore, the multiple enzyme approach was the best (37%) but cellulase also gave a good result.

The biomass removal results in the MFR experiment agreed essentially with 37% removal between 72.5 and 96.5 hr (Figure 4). The pressure drop data (Figure 5) in the same (MFR) experiment support this finding.

Example III

Focusing on the mixed enzyme composition of alpha-amylase, beta-glucanase and protease, further MFR studies were conducted to determine the effect of pH on its effectiveness in biofilm removal. The mixed composition was tested using a polyol biodispersant as a control in single-cycle synthetic tap water with pH maintained at 7.5, 8.5 or 9.0. The results are summarized in Table III. The composition was effective up to pH 9. The efficacy of the composition is also compared to that of the dispersant at neutral and alkaline pH's in Table III.

Example IV

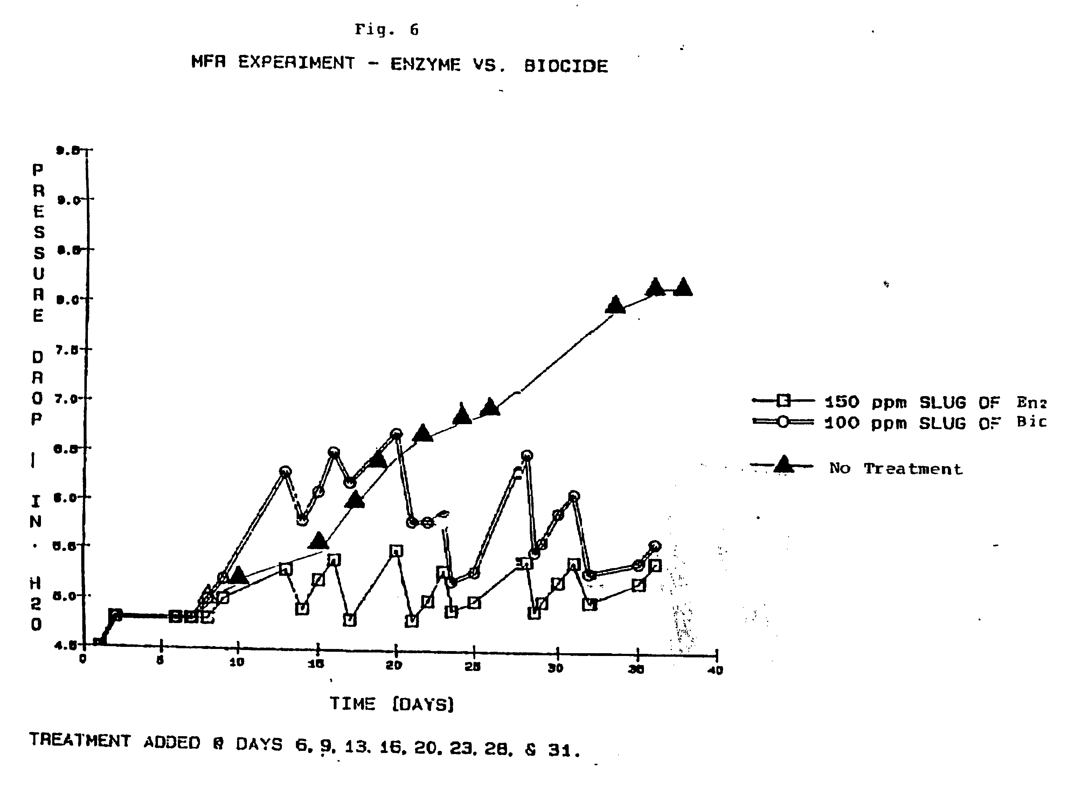

An experiment was run on the Microbial Fouling Reactor and the results are shown in Figure 6. The experiment was designed to test whether the mixed enzyme composition would keep a surface clean. The conditions for the experiment differed in substrate concentration and treatment dosage (Table IV). The substrate concentration was low, similar to substrate level in cooling water. The dose was either a slug of biocide or enzyme product.

In Figure 6, the control or no treatment (-▲-) curve indicates what biofilm growth is possible in low substrate conditions. The biocide curve indicates 100 ppm nonoxidizing biocide slugged in the reactor at days 6, 9, 13, 16, 20, 23, 28 and 31 caused losses in biofilm, as measured by decreases in pressure drop. The curve representing performance of the enzyme combination also indicates biofilm loss after each treatment. After 31 days the difference between the biocide-treated line and the untreated control was 2.4 inches (6.1 cm) $H_2O$ ($\Delta$ p = 2.4 in. (6.1 cm)); 2.7 inches (6.8 cm) $H_2O$ using the enzyme blend. Figure 6 indicates that after treatments were stopped, the biofilm in both lines grew.

The results were good. The experiment demonstrated that the enzymes controlled the biofilm growth very well over one month. The enzyme blend, which is nontoxic, performed at least as well as the toxicant (nonoxidizing) biocides.

In the specification and claims glucanase is equivalent and equal to beta-glucanase.

TABLE I

| Biofilm Removal Test Conditions | | |
|---|---|---|
| Parameter | Conditions Per Apparatus | |
| | BRR | MFR |
| pH | 8.5 | 7.5, 8.5 or 9.0[a] |
| Temperature (°C) | 36±1[b] | 33.0±1[c] |
| Make-up Water | Synthetic Chicago Tap[d] | Synthetic Chicago Tap[d] |
| Substrate Concentration | | |
| TSB | 50 ppm | 50 ppm |
| D-glucose | 50 ppm | 50 ppm |
| Inoculum | Field Composite | Field Composite |
| Growth Period | 72 Hr | p ≤ 10 in[e] |
| Treatment | | |
| Enzyme Concentration | 100 ppm | 100 ppm |
| Duration | 24 Hr | 24 Hr |

a pH setting depended on experiment.
b BRR temperature is consistently 36° ±1° C resulting from operation of recirculating pump.
c MFR temperature is thermostatically controlled.
d Synthetic Chicago tap water.
e MFR p of 10 inches occurred at approximately 72 Hr.

TABLE II

| Summary of BRR Studies at pH 8.5 | |
|---|---|
| Type of Enzyme | % Removal |
| Neutral Protease | -10.0 |
| Alkaline Protease (1) | -50.0 |
| Alkaline Protease (2) | 18.0 |
| Debranching Enzyme | -19.0 |
| Alkaline alpha-amylase | 21.5 |
| Beta-glucanase (1) | 0 |
| Beta-glucanase (2) | 14.0 |
| Cellulase[a] | 23.0 |
| Alpha-amylase, Beta-glucanase + Protease[b] | 37.0 |

a Cellulase attacks the beta-linkage between sugar molecules.
b Alpha-amylase, beta-glucanase and neutral protease activities.

TABLE III

| Effect of pH on Enzyme Treatment Performance[a] | | |
|---|---|---|
| | % Removal of Biofilm[b] | |
| pH | Enzyme | Biodispersant |
| 7.5 | 48 | 3 |
| 8.5 | 35[b] | 1[b] |
| 9.5 | 44[b] | 11[b] |

a Performance is evaluated at 100 ppm enzyme, 20 ppm biodispersant concentration levels.
b Removal is an average of two experiments at 33 ± 1° C.

TABLE IV

| Microbial Fouling Reactor Test Conditions for Biofilm Control Experiment | |
|---|---|
| Parameter | Conditions |
| pH | 8.5 |
| Temperature | 33.0±1.0°C |
| Make-up Water | Synthetic Tap Water |
| Substrate Concentration | |
| TSB | 10 ppm |
| D-glucose | 10 ppm |
| Inoculum | Field Composite |
| Treatments | |
| Enzyme Concentration | 150 ppm |
| Duration | Slug dose |
| Frequency | Twice per week |
| Biocide Concentration | 100 ppm |
| Duration | Slug dose |
| Frequency | Twice per week |

**Claims**

1. A method of preventing the build-up of slime or removing slime from surfaces contacted with industrial water which comprises contacting said surfaces with an effective enzymatic amount of cellulase and/or beta-glucanase.

2. A method of preventing the build-up of slime on removing slime from surfaces contacted with industrial water which comprises contacting said surfaces with an effective amount of a preparation including beta-glucanase, alpha-amylase and protease, in which the beta-glucanase may be wholly or partially replaced by cellulase.

3. A method according to claim 2 wherein the preparation includes 2 parts beta-glucanase, 0.5 to 1 part protease and 1 to 2 part alpha-amylase.

4. A method according to claim 3 wherein the beta-glucanase is partially replaced by cellulase.

5. A method according to claim 1 which comprises treating said water with at least 80 ppm of beta-glucanase, the active ingredient degrading the slime being the beta-glucanase.

6. A method according to any one of claims 2 to 4 wherein the composition is present at a concentration of at least 2 ppm in the industrial water.

7. Use of a composite enzyme system consisting of beta-glucanase, alpha-amylase and protease to digest microbial slime and reduce microbial attachment and biofilm.

8. Use of cellulase to digest microbial slime and reduce microbial attachment and biofilm.

9. A use according to claim 7 wherein the enzyme system consists of 2 parts beta-glucanase, at least 1 and less than 2 parts alpha-amylase and .5 to 1 part protease.

EP 0 388 115 A1

BIOFILM
TEST SECTIONS

ROTAMETER

INFLUENT

VALVE

MIXING
CHAMBER

PUMP

OVERFLOW

FIGURE 1     Biofilm Removal Reactor System

FIGURE 2:

BIOFILM REMOVAL TEST
SINGLE CYCLE SYNTHETIC $H_2O$--pH 8.5

EP 0 388 115 A1

CONTROL (BLANK)
BIODISPERSANT
CELLULASE
$\alpha$-amylase + $\beta$-glucanı
+ protease (1:2:1 rat

TIME [HR]

INFLUENT (MAKEUP, SUBSTRATE, TEST SOLUTIONS, ETC.)

PH CONTROL

TEMP CONTROL

MICROSCOPE

FERMENTER

SAMPLE TEST SECTION

VALVE

FLOW METER

OVERFLOW

GLASS SECTION

PRESSURE DROP SECTION

PUMP

DIFFERENTIAL PRESSURE GUAGE

EP 0 388 115 A1

FIGURE 3 Microbial Fouling Reactor (MFR)

FIGURE 4

BIOFILM MASS VS. TIME
ENZYME AND BIODISPERSANT @ pH 8.5

TIME (IN HR)

BIOMASS mg/cm$^2$

ENZYME (100 PPM @ 72.5 H.)

BIODISPERSANT (20 PPM @ 72.5 H.)

10 ppm Cl$_2$ added at 96.5 H

EP 0 388 115 A1

FIGURE 5
PRESSURE DROP VS. TIME
ENZYME AND BIODISPERSANT @ pH 8.5

Fig. 6

MFR EXPERIMENT — ENZYME VS. BIOCIDE

TREATMENT ADDED @ DAYS 6, 9, 13, 16, 20, 23, 28, & 31.

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| X | DE-A-3 741 583 (KEMIRA OY) <br> * Page 2, claims * | 1 | C 02 F 1/50 |
| A | | 2,5-7 | |
| A,D | EP-A-0 129 315 (ECONOMICS LABORATORY INC.) <br> * Page 4, lines 21-32; page 5, line 33 - page 6, line 15 * | 2,6,7 | |
| A | PATENT ABSTRACTS OF JAPAN, vol. 12, no. 491 (C-554)[3338], 21st December 1988, page 76 C 554; & JP-A-63 202 677 (MITSUBISHI YUKA BADISCHE CO., LTD) 22-08-1988 | 1,7 | |

**TECHNICAL FIELDS SEARCHED (Int. Cl.5)**

C 02 F

**The present search report has been drawn up for all claims**

| Place of search | Date of completion of the search | Examiner |
|---|---|---|
| THE HAGUE | 25-06-1990 | GONZALEZ ARIAS M.L. |

EPO FORM 1503 03.82 (P0401)